**Europäisches Patentamt**

**European Patent Office**

**Office européen des brevets**

(19)

(11) **EP 1 402 852 A1**

(12) **EUROPEAN PATENT APPLICATION**

(43) Date of publication:
**31.03.2004 Bulletin 2004/14**

(51) Int Cl.$^7$: **A61F 2/16**

(21) Application number: **03021678.2**

(22) Date of filing: **29.09.2003**

(84) Designated Contracting States:
**AT BE BG CH CY CZ DE DK EE ES FI FR GB GR HU IE IT LI LU MC NL PT RO SE SI SK TR**
Designated Extension States:
**AL LT LV MK**

(30) Priority: **30.09.2002 JP 2002288137**

(71) Applicant: **NIDEK CO., LTD**
**Gamagori-shi, Aichi 443-0035 (JP)**

(72) Inventors:
- **Sumiya, Toshifumi**
  **Kota-cho Nukata-gun Aichi-ken 444-0116 (JP)**
- **Murakami, Naho**
  **Hoi-gun Aichi-ken 441-0203 (JP)**

(74) Representative:
**Winter, Brandl, Fürniss, Hübner, Röss, Kaiser,**
**Polte Partnerschaft**
**Patent- und Rechtsanwaltskanzlei**
**Alois-Steinecker-Strasse 22**
**85354 Freising (DE)**

(54) **Aspherical intraocular lens**

(57)     An intraocular lens (1) having a certain accommodation range while maintaining favorable contrast. The intraocular lens (1), arranged inside an eye after a crystalline lens is removed therefrom, has an optical part (2) including anterior (2a) and posterior (2b) refractive surfaces, the optical part having refractive power, wherein at least one of the refractive surfaces of the optical part (2) has an aspherical shape so as to have greater depth of focus than depth of focus in a case where the optical part (2) is a spherical lens.

FIG. 1 A

EP 1 402 852 A1

FIG. 1 B

**EP 1 402 852 A1**

**Description**

BACKGROUND OF THE INVENTION

1. Field of the Invention

The present invention relates to an intraocular lens which is arranged inside an eye in cataract surgery.

2. Description of Related Art

Conventionally, in cataract surgery, a crystalline lens developing cataract is absorbed and removed through phacoemulsification, and an intraocular lens is arranged to replace the removed crystalline lens. The intraocular lens used in this kind of surgery is often a single focal lens. On the other hand, a multifocal ophthalmologic lens is proposed which has an aspherical (non-spherical) shape in which a part having refractive power for far vision and that having refractive power for near vision are concentrically formed so as to be in focus for both the far and near visions. (For example, see Japanese Patent Application Unexamined Publication No. Heil-154119 corresponding to U.S. Patent No. 4,881,804.)

However, when the single focal intraocular lens which is fixed-focus and has constant refractive power is used, accommodation function of an eyeball is lost, and only an object at a certain distance is brought into focus. Besides, though the multifocal ophthalmologic lens focuses on both the far and near visions, an image of the far vision and that of the near vision are simultaneously formed inside the eyeball, and a light bundle entered the eyeball is divided for the far vision and the near vision, so that light intensity of one of those images is reduced and contrast is lowered.

SUMMARY OF THE INVENTION

An object of the invention is to overcome the problems described above and to provide an intraocular lens having a certain accommodation range while maintaining favorable contrast.

To achieve the objects and in accordance with the purpose of the present invention, an intraocular lens has an optical part including anterior and posterior refractive surfaces, the optical part having refractive power, wherein at least one of the refractive surfaces of the optical part has an aspherical shape so as to have greater depth of focus than depth of focus in a case where the optical part is a spherical lens.

Additional objects and advantages of the invention are set forth in the description which follows, are obvious from the description, or may be learned by practicing the invention. The objects and advantages of the invention may be realized and attained by the intraocular lens in the claims.

BRIEF DESCRIPTION OF THE DRAWINGS

The accompanying drawings, which are incorporated in and constitute a part of this specification, illustrate embodiments of the present invention and, together with the description, serve to explain the objects, advantages and principles of the invention. In the drawings,

Figs. 1A and 1B are schematic views showing an outer appearance of an intraocular lens consistent with one preferred embodiment;
Fig. 2 is a schematic sectional view showing a model eye;
Fig. 3A is a view showing an example in which the intraocular lens is arranged inside the model eye to replace a crystalline lens;
Fig. 3B is a view showing longitudinal aberration of a whole eyeball in which the intraocular lens shown in Fig. 3A is arranged;
Fig. 4 is a view showing the longitudinal aberration of the whole eyeball in a case where the aberration is small;
Fig. 5 is a view showing the longitudinal aberration of the whole eyeball in a case where the aberration is medium;
Fig. 6 is a view showing the longitudinal aberration of the whole eyeball in a case where the aberration is large;
Fig. 7 is a view showing change in a light bundle diameter in an optical axis direction of the respective lenses with the different aberrations; and
Fig. 8 is a view showing a ratio of the light bundle diameter in the optical axis direction to a minimum light bundle diameter.

DETAILED DESCRIPTION OF THE PREFERRED EMBODIMENTS

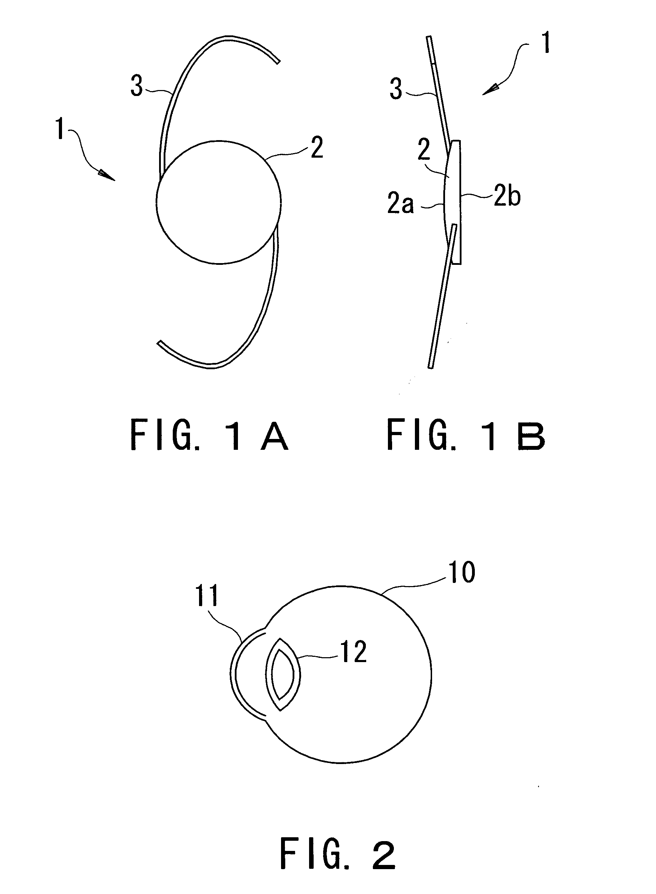

A detailed description of one preferred embodiment of an intraocular lens embodied by the present invention is provided below with reference to the accompanying drawings. Fig. 1A is a view showing the intraocular lens of the preferred embodiment consistent with the present invention when viewed from above, and Fig. 1B is a view showing the intraocular lens in Fig. 1A when viewed from its side. An intraocular lens 1 consistent with the preferred embodiment includes an optical part 2 in a plane-convex lens shape and a supporting member 3 for fixing and supporting (holding) the optical part 2 inside the eye. In the optical part 2, an anterior refractive surface (a surface on a cornea side when the intraocular lens 1 is arranged inside the eye) 2a has an aspherical shape, and a posterior refractive surface (a surface on a fundus side when the intraocular lens 1 is arranged inside the eye) 2b has a plane shape. The anterior refractive surface 2a of the optical part 2 has the aspherical shape so that the optical part 2 has greater depth of focus than a conventional intraocular lens having a spherical shape (a spherical intraocular lens). In other words, the anterior refractive surface 2a of the optical part 2 has the aspherical shape so that the optical part 2 has larger spherical aberration than the conventional spherical intraocular lens.

For this reason, in the intraocular lens 1, a light bundle diameter comes to have little difference with a minimum light bundle diameter at a focal point of the optical part 2 in a certain range of an optical axis direction position. Since the conventional single focal spherical intraocular lens has the spherical surface, if it slightly goes out of focus, a difference between a light bundle diameter at a point out of focus and a minimum light bundle diameter at a point in focus (a focal point) becomes great, and resolution is greatly lowered (a difference in the resolution becomes great). On the other hand, in the intraocular lens 1, if it slightly goes out of focus, since the difference between the light bundle diameter at the point out of focus and the minimum light bundle diameter at the focal point is little as compared to the conventional single focal spherical intraocular lens, the resolution is not so lowered (the difference in the resolution is little) . That is to say, the intraocular lens 1 has a larger accommodation range than the conventional single focal spherical intraocular lens.

For example, it is assumed that a wearer may quite normally accept reduction in resolution ranging up to approximately 10% relative to the resolution at the focal point (as in a range perceived to be in focus). In the conventional single focal spherical intraocular lens, the range from a focal point at which the minimum light bundle diameter is obtained to an optical axis direction position at which the light bundle diameter marks an approximate 10% rise is narrow, so a range perceived to be in focus is narrow. On the other hand, the intraocular lens 1 may have a range, preferably, approximately not less than two times, more preferably, approximately three times the range to the optical axis direction position at which the light bundle diameter marks an approximate 10% rise with respect to the minimum light bundle diameter at the focal point in the conventional single focal spherical intraocular lens.

Incidentally, it is preferable that such range is as large as possible. However, if the range is too large, the minimum light bundle diameter at the focal point becomes too large, and sufficient resolution becomes hard to be obtained even at the point in focus. Therefore, the aspherical shape of the anterior refractive surface 2a of the optical part 2 may be determined so as to have the minimum light bundle diameter, preferably, approximately not more than four times, more preferably, approximately not more than three times the minimum light bundle diameter in the case of the conventional single focal spherical intraocular lens. If the minimum light bundle diameter becomes approximately not less than four times the minimum light bundle diameter in the case of the conventional single focal spherical intraocular lens, sufficient resolution becomes hard to be obtained.

As a material for the optical part 2, acrylic resin such as polymethyl methacrylate (PMMA) or polyhydroxy ethyl methacrylate (PHEMA) which is hydrous and in which distance between molecules is relatively long, silicone resin and the like, which are materials generally used for the optical part of the intraocular lens, may be used. For the supporting member 3, a material such as PMMA generally used for the supporting member of the intraocular lens may be used.

The intraocular lens 1 as above may be obtained by an existent intraocular lens production method such as a method of polymerizing and hardening a base material put in a molding box having a predetermined intraocular lens shape, or that of cutting a base material having a plate shape into a predetermined intraocular lens shape.

As a formula for determining refractive power of the intraocular lens 1 (the optical part 2), an existent formula such as an SRK formula or a BINKHORST formula which are conventionally used may be employed. For example, the SRK formula is expressed as Formula 1.

(Expression 1)

$$IOL = A - 2.5 \times AL - 0.9 \times K - DR \times (0.0875 \times A - 8.55) \qquad \text{(Formula 1)}$$

In Formula 1, K indicates corneal refractive power (D), AL indicates an axial length (mm), A indicates an A constant, and DR indicates desired refractive power (D) for a corrective lens after surgery.

Further, as for a design of a lens curve for preparing an aspherical lens, ray tracing may be performed concentrically from an optical center to determine a desired curved shape. At this time, information on corneal aberration and the like of the wearer are obtained, and the curved shape of the anterior refractive surface 2a of the intraocular lens 1 is determined in consideration of that information, so that a more accurate aspherical shape may be designed. The information on the corneal aberration may be obtained by using a conventional ophthalmic apparatus such as a corneal shape measurement apparatus.

Hereinafter, change in the resolution (change in the light bundle diameter), a range of accommodative power and the like due to a difference in the curved shape of the refractive surface (a difference in the aberration) of the optical part of the intraocular lens will be described. For information, the aspherical shape of the intraocular lenses named below is an axisymmetric rotating aspherical shape. Only a conic constant of the aspherical shape is changed to vary magnitude of the spherical aberration, and a base curve (radius of curvature at an apex) and an aspherical factor are not changed.

Fig. 2 is a schematic sectional view showing a model eye. A Gullstrand's model eye 10 is used as a human eyeball model, and is provided with a cornea model 11 and a crystalline lens model 12. Table 1 provides data on each part in the model eye 10.

(Table 1)

|  | Radius of curvature (mm) | Distance (mm) | Refractive index |
|---|---|---|---|
| Corneal anterior surface | 7.7 | 0.5 | 1.376 |
| Corneal posterior surface | 6.8 | 3.1 | 1.336 |
| Crystalline lens anterior surface | 10.0 | 0.546 | 1.386 |
| Lens nucleus anterior surface | 7.911 | 2.419 | 1.406 |
| Lens nucleus posterior surface | -5.76 | 0.635 | 1.386 |
| Crystalline lens posterior surface | -6.0 | 16.8 | 1.336 |
| Retina | - | - | - |

Using the model eye 10 with these characteristics, the intraocular lens having the spherical shape or that having the aspherical shape are variously interchanged with the crystalline lens model 12 to obtain change in a diameter of a light bundle in the optical axis direction (a spot size hereinafter), wherein a parallel light bundle passed through the cornea model 11 and the intraocular lens is collected to form the light bundle.

<Aspherical lens (0 aberration)>

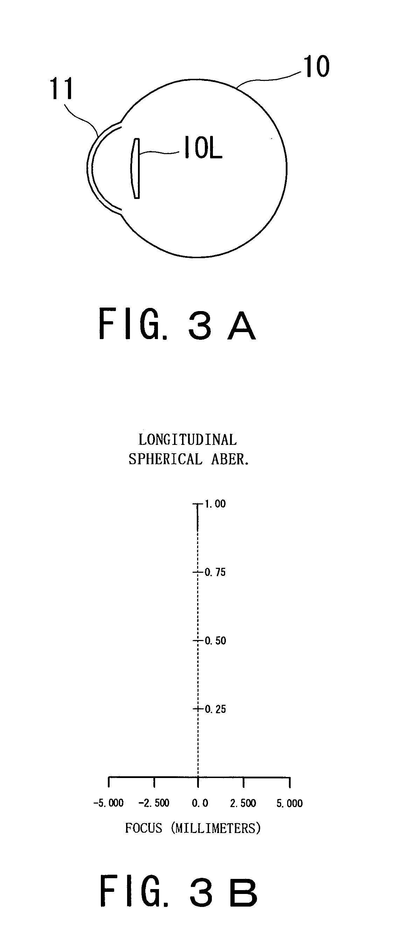

Fig. 3A is a view showing an example in which an intraocular lens having an aspherical shape on one refractive surface (an aspherical intraocular lens in a plane-convex lens shape) is arranged to replace the crystalline lens model 12 inside the model eye 10 shown in Fig. 2. (In Fig. 3A, the supporting member 3 is omitted from illustration.)

In addition, Fig. 3B is a view showing longitudinal aberration of the whole eyeball including the intraocular lens shown in Fig. 3A. A vertical axis indicates a distance (mm) from the optical center, and a horizontal axis indicates a position in the optical axis direction (mm). Therein, a minus side of the horizontal axis is an intraocular lens side (an anterior side).

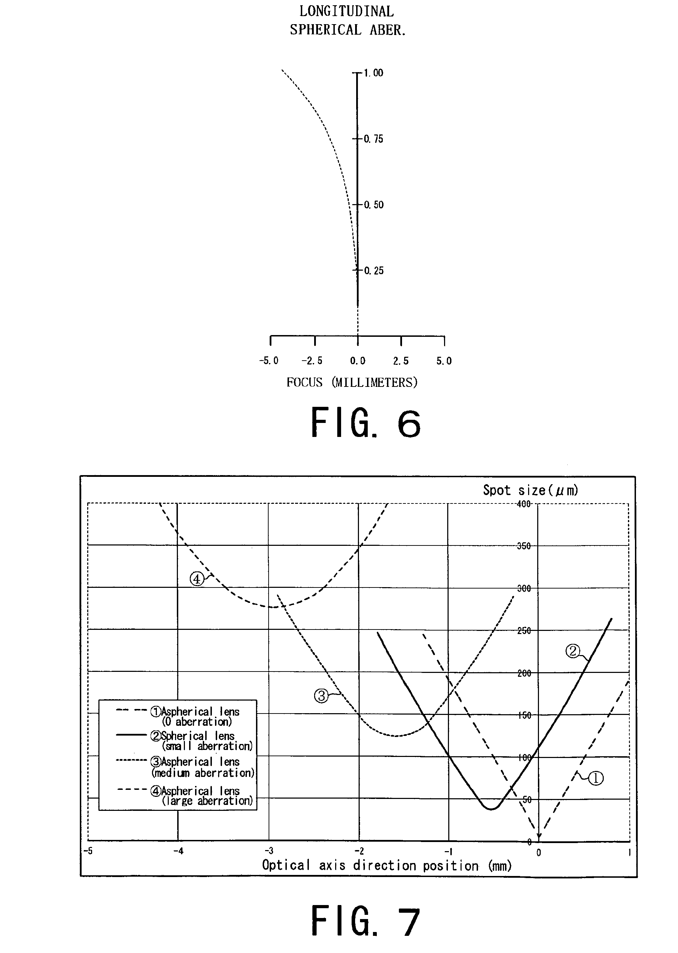

As shown in Fig. 3B, this intraocular lens is designed so that the aberration of the optical system of the whole eyeball becomes 0. By using this intraocular lens, change in the spot size in the optical axis direction when the parallel light bundle enters the eye was obtained. The results are shown in Fig. 7. In Fig. 7, a vertical axis indicates the spot size, and a horizontal axis indicates a position in the optical axis direction. Therein, an origin point of the horizontal axis is a position of the minimum spot size in the case of the aberration being 0 (a Gauss image surface here). The minus side of the horizontal axis is the intraocular lens side (the anterior side) with respect to the position of the minimum spot size in the case of the aberration being 0.

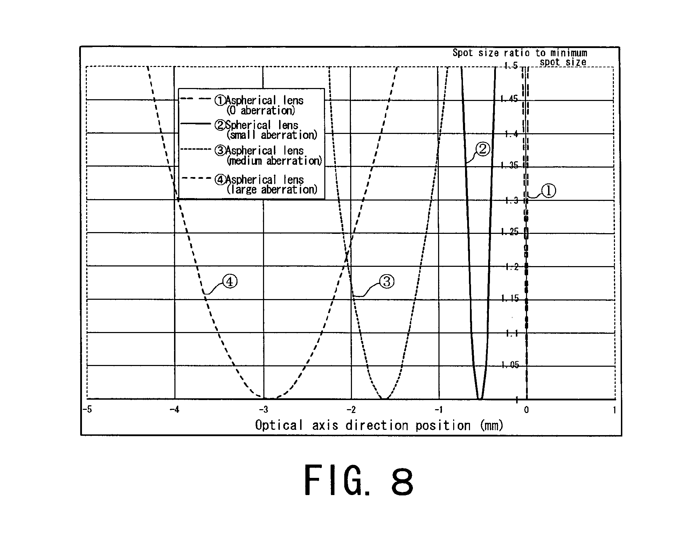

Further, Fig. 8 is a view showing a ratio of the spot size to the minimum spot size shown in Fig. 7. Therein, a vertical axis indicates the ratio to the minimum spot size, and a horizontal axis indicates the position in the optical axis direction.

As shown in Figs. 7 and 8, when the spherical aberration is approximately 0, the spot size minimizes at the origin point, and the ratio to the minimum spot size marks a 10% or less rise in a range of the order of 0.02 mm which extends from -0.01 mm to +0.01 mm in the optical axis direction from the position of the minimum spot size. For this reason, there is almost no range where an approximately same spot size as the minimum spot size is maintained in the optical axis direction.

<Spherical lens (small aberration)>

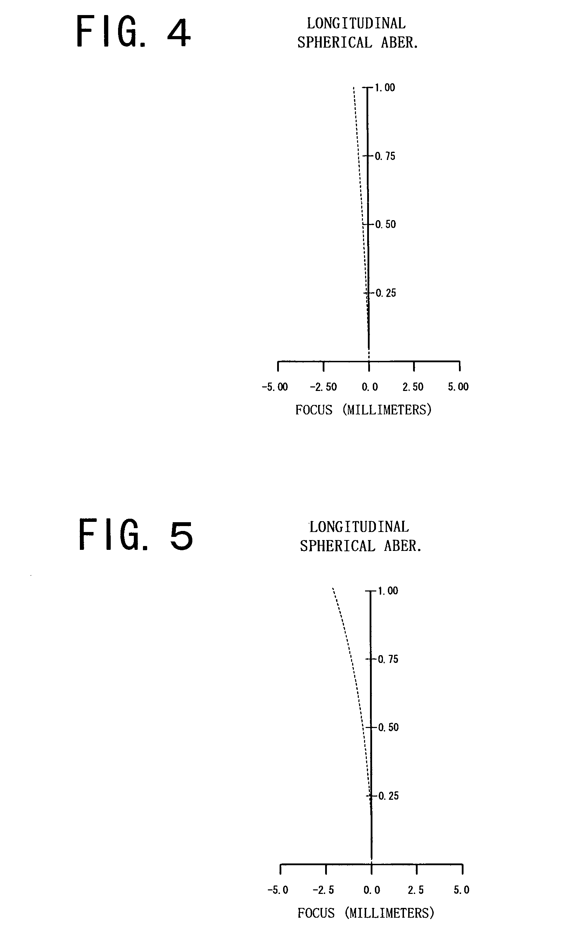

Fig. 4 is a view showing the longitudinal aberration of the whole eyeball when an intraocular lens in the spherical shape is arranged inside the model eye 10. The intraocular lens used here is a conventionally used single focal intraocular lens having a spherical shape (a spherical intraocular lens in a plane-convex lens shape) , which has data on each part as provided in Table 2. As shown in Fig. 4, in this intraocular lens, the spherical aberration exists in the minus side though it is small.

(Table 2)

|  | Radius of curvature (mm) | Distance (mm) | Refractive index |
|---|---|---|---|
| Corneal anterior surface | 7.7 | 0.5 | 1.376 |
| Corneal posterior surface | 6.8 | 3.1 | 1.336 |
| Intraocular lens anterior surface | 9.0 | 1.12 | 1.492 |
| Intraocular lens posteriorsurface | ∞ | 19.28 | 1.336 |
| Retina | - | - | - |

As described before, Figs. 7 and 8 show the change in the spot size and that in the spot size ratio. As shown in Figs. 7 and 8, when the spherical aberration slightly exists, the spot size almost minimizes in the vicinity of a -0.54 mm position, and the ratio to the minimum spot size marks a 10% or less rise in a range of the order of 0.17 mm which extends from -0.62 mm to -0.45 mm. Generally, a 1 mm change in the axial length corresponds to about 3D (diopter). Therefore, when that range (width) is converted to diopter, it corresponds to about 0.5D, and the accommodation may be performed in this range. Incidentally, both in Figs. 7 and 8, the minimum spot size is not aligned to be positioned at the origin point because a deviation amount of the focal point presented when "the aspherical lens (0 aberration)" is taken as a reference is indicated without alignment.

<Aspherical lens (medium aberration)>

Fig. 5 is a view showing the longitudinal aberration of the whole eyeball when an intraocular lens in an aspherical shape (an aspherical intraocular lens in a plane-convex lens shape) consistent with the preferred embodiment, which has larger spherical aberration than "the spherical lens (small aberration)", is arranged inside the model eye 10. As shown in Fig. 5, this intraocular lens has medium aberration which is larger than "the spherical lens (small aberration)".

As described before, the change in the spot size and that in the spot size ratio are shown in Figs. 7 and 8. As shown in Figs. 7 and 8, when the spherical aberration exists to a medium degree, the spot size almost minimizes in the vicinity of a -1.61 mm position, and the ratio to the minimum spot size marks a 10% or less rise in a range of the order of 0.54 mm which extends from -1.88 mm to -1.34 mm. The range (width) is converted to diopter of about 1.6D, and an accommodation range not less than three times "the spherical lens (small aberration)" is obtained. However, the minimum spot size becomes larger to be approximately three point four times "the spherical lens (small aberration)". Assuming that the wearer has visual acuity of the order of 1.5 in the case of using "the spherical lens (small aberration)", visual acuity of the order of 0.4 is to be obtained with "the intraocular lens in an aspherical shape (medium aberration)". Even if the visual acuity is 0.4, daily life is not disturbed, and in the case of driving a car and the like, the curved shape of the intraocular lens may be determined so that the visual acuity of the order of 0.7 is obtained. In this case, as the resolution is obtained, an accommodable range narrows. However, when compared to the conventionalintraocularlenshaving thesphericalshape, a larger accommodation range is obtained. Further, since this kind of single focal intraocular lens having the aspherical shape has no remarkable reduction in light intensity, the contrast is prevented from lowering, and a higher contrast than the multifocal intraocular lens may be maintained.

<Aspherical lens (large aberration)>

Fig. 6 shows the longitudinal aberration of the whole eyeball when an intraocular lens in an aspherical shape (an aspherical intraocular lens in a plane-convex lens shape), which has larger spherical aberration than "the aspherical lens (medium aberration)", is arranged inside the model eye 10. As shown in Fig. 6, this intraocular lens has larger spherical aberration than "the aspherical lens (medium aberration)".

As described before, the change in the spot size and that in the spot size ratio is shown in Figs. 7 and 8. As shown in Figs. 7 and 8, in the case of the large spherical aberration, the spot size almost minimizes in the vicinity of a -2.91 mm position, and the ratio to the minimum spot size marks a 10% or less rise in a range of the order of 1.1 mm which extends from -3.45 mm to -2.35 mm. This range (width) is converted to diopter of about 3.3D, and an accommodation range not less than six times "the spherical lens (small aberration)" is obtained. However, the minimum spot size further becomes larger to be approximately seven point six times "the spherical lens (small aberration)". Assuming that the visual acuity of the order of 1.5 is obtained in a case where the wearer uses "the spherical lens (small aberration)", "the aspherical shape (large aberration)" is impracticable because only the visual acuity of the order of 0.2 is obtained, and the reduction in the visual acuity becomes larger.

Incidentally, in the preferred embodiment described above, the anterior refractive surface of the optical part 2 has the aspherical shape. However, the posterior spherical surface may have the aspherical shape, and both the surfaces may have the aspherical shape. In addition, the optical part 2 has the plane-convex lens shape; however, the present invention is not limited thereto. The optical part 2 may have a both-convex lens shape or a convex meniscus lens shape.

Besides, what is described above is a relation between the spherical aberration when the intraocular lens is inserted into the Gullstrand's model eye and the change in the light bundle diameter in the optical axis direction due to the spherical aberration. As for an actual eyeball, because the corneal curvature and the axial length are individually different, the spherical aberration and the change in the light bundle diameter in the optical axis direction are also individually different. Therefore, the corneal curvature, the axial length and the like may be previously obtained by using the ophthalmic apparatus such as the corneal shape measurement apparatus and an axial length measurement apparatus, and measurement data thus obtained are replaced with data on each part of the Gullstrand's model eye described above to control the spherical aberration in the optical system of the whole eyeball, so that more accurate correction may be performed.

As described above, according to the present invention, a certain accommodation range is obtained while maintaining favorable contrast even if the intraocular lens is single focal.

The foregoing description of the preferred embodiments of the invention has been presented for purposes of illustration and description. It is not intended to be exhaustive or to limit the invention to the precise form disclosed, and modifications and variations are possible in the light of the above teachings or may be acquired from practice of the invention. The embodiments chosen and described in order to explain the principles of the invention and its practical application to enable one skilled in the art to utilize the invention in various embodiments and with various modifications as are suited to the particular use contemplated. It is intended that the scope of the invention be defined by the claims appended hereto.

## Claims

1. An intraocular lens (1) arranged inside an eye after a crystalline lens is removed therefrom, the intraocular lens comprising:

    an optical part (2) including anterior and posterior refractive surfaces (2a,2b), the optical part having refractive power;

    **characterized by**
    at least one of the refractive surfaces of the optical part having an aspherical shape so as to have greater depth of focus than depth of focus in a case where the optical part is a spherical lens.

2. The intraocular lens according to claim 1, wherein at least one of the refractive surfaces of the optical part has the aspherical shape so that, when a parallel light bundle enters the eye, a range of an optical axis direction position, at which a light bundle diameter marks a 10% or less rise with respect to a minimum light bundle diameter at a focal point of the optical part, is not less than two times a range in a case where the optical part is the spherical lens.

3. The intraocular lens according to any one of claims 1 and 2, wherein at least one of the refractive surfaces of the optical part has the aspherical shape so that, when the parallel light bundle enters the eye, the minimum light bundle diameter at the focal point of the optical part becomes not more than four times the minimum light bundle diameter in a case where the optical part is the spherical lens.

4. The intraocular lens according to any one of claims 1 to 3, wherein at least one of the refractive surfaces of the optical part has the aspherical shape so that, when the parallel light bundle enters the eye, the range of the optical axis direction position, at which the light bundle diameter marks a 10% or less rise with respect to the minimum light bundle diameter at the focal point of the optical part, is three times the range in a case where the optical part is the spherical lens.

5. The intraocular lens according to any one of claims 1 to 4, wherein at least one of the refractive surfaces of the optical part has the aspherical shape so that, when the parallel light bundle enters the eye, the minimum light bundle diameter at the focal point of the optical part becomes not more than three times the minimum light bundle diameter in a case where the optical part is the spherical lens.

FIG. 1 A    FIG. 1 B

FIG. 2

# FIG. 3 A

LONGITUDINAL
SPHERICAL ABER.

1.00

0.75

0.50

0.25

-5.000    -2.500    0.0    2.500    5.000

FOCUS (MILLIMETERS)

# FIG. 3 B

# FIG. 4

LONGITUDINAL
SPHERICAL ABER.

FOCUS (MILLIMETERS)

# FIG. 5

LONGITUDINAL
SPHERICAL ABER.

FOCUS (MILLIMETERS)

LONGITUDINAL
SPHERICAL ABER.

FIG. 6

FIG. 7

FIG. 8

EP 1 402 852 A1

## EUROPEAN SEARCH REPORT

**European Patent Office**

Application Number

EP 03 02 1678

### DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X | US 5 796 462 A (ROFFMAN JEFFREY H ET AL) 18 August 1998 (1998-08-18) * column 2, line 46 - column 3, line 3 * * claims 1,9-11,18; figures * --- | 1-5 | A61F2/16 |
| A | EP 0 472 291 A (JOHNSON & JOHNSON VISION PROD) 26 February 1992 (1992-02-26) * column 5, line 13 - column 6, line 44 * * claims 1,8,9,13; figures 3,4,7 * * column 8, line 1 - column 9, line 5 * --- | 1-5 | |
| A | US 5 864 378 A (PORTNEY VALDEMAR) 26 January 1999 (1999-01-26) * column 1, line 50 - line 61 * * column 3A, line 2 - line 18 * * column 3, line 50 - column 4, line 20 * * column 5, line 32 - line 38 * * column 6, line 65 - column 7, line 13 * * claims; figures 3,4 * --- | 1-5 | |
| A | WO 01 84216 A (ALLERGAN SALES INC) 8 November 2001 (2001-11-08) * page 5, line 8 - line 18 * * claims; figures * * page 6, line 8 - line 11 * --- | 1-5 | TECHNICAL FIELDS SEARCHED (Int.Cl.7) A61F |
| A | US 4 769 033 A (NORDAN LEE T) 6 September 1988 (1988-09-06) * column 3, line 24 - line 36 * * claims; figure 3 * --- | 1-5 | |
| A | EP 0 503 111 A (KASHIWAGI TOYOHIKO) 16 September 1992 (1992-09-16) * column 1, line 53 - column 2, line 2 * * claims; figures * --- | 1-5 | |
|  | -/-- | | |

The present search report has been drawn up for all claims

| Place of search | Date of completion of the search | Examiner |
|---|---|---|
| BERLIN | 28 January 2004 | Kuehne, H-C |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

14

**European Patent Office**

## EUROPEAN SEARCH REPORT

Application Number

EP 03 02 1678

| | DOCUMENTS CONSIDERED TO BE RELEVANT | | |
|---|---|---|---|
| Category | Citation of document with indication, where appropriate, of relevant passages | Relevant to claim | CLASSIFICATION OF THE APPLICATION (Int.Cl.7) |
| A | US 5 019 098 A (MERCIER JEAN-LOUIS) 28 May 1991 (1991-05-28) * column 6, line 35 - line 41 * * claims 1-5; figure 3 * --- | 1-5 | |
| A | US 5 888 122 A (GUPTA AMITAVA ET AL) 30 March 1999 (1999-03-30) * column 7, line 7 - line 16 * * figure 7 * --- | 1-5 | |
| A | US 5 173 723 A (VOLK DONALD A) 22 December 1992 (1992-12-22) * claims; figures * ----- | 1-5 | |

TECHNICAL FIELDS
SEARCHED      (Int.Cl.7)

The present search report has been drawn up for all claims

| Place of search | Date of completion of the search | Examiner |
|---|---|---|
| BERLIN | 28 January 2004 | Kuehne, H-C |

EPO FORM 1503 03.82 (P04C01)

**ANNEX TO THE EUROPEAN SEARCH REPORT
ON EUROPEAN PATENT APPLICATION NO.**

EP 03 02 1678

This annex lists the patent family members relating to the patent documents cited in the above-mentioned European search report.
The members are as contained in the European Patent Office EDP file on
The European Patent Office is in no way liable for these particulars which are merely given for the purpose of information.

28-01-2004

| Patent document cited in search report | | Publication date | Patent family member(s) | | Publication date |
|---|---|---|---|---|---|
| US 5796462 | A | 18-08-1998 | AU | 707422 B2 | 08-07-1999 |
| | | | AU | 5206796 A | 14-11-1996 |
| | | | BR | 9602168 A | 13-01-1998 |
| | | | CA | 2175653 A1 | 05-11-1996 |
| | | | CN | 1164652 A ,B | 12-11-1997 |
| | | | EP | 0742461 A2 | 13-11-1996 |
| | | | JP | 9021905 A | 21-01-1997 |
| | | | NO | 961803 A | 05-11-1996 |
| | | | NZ | 286484 A | 19-12-1997 |
| | | | SG | 46732 A1 | 20-02-1998 |
| | | | ZA | 9603546 A | 03-11-1997 |
| EP 0472291 | A | 26-02-1992 | US | 5050981 A | 24-09-1991 |
| | | | AT | 128248 T | 15-10-1995 |
| | | | AU | 636502 B2 | 29-04-1993 |
| | | | AU | 8117291 A | 30-01-1992 |
| | | | BR | 9102977 A | 11-02-1992 |
| | | | CA | 2047507 A1 | 25-01-1992 |
| | | | CN | 1058474 A ,B | 05-02-1992 |
| | | | CZ | 9102295 A3 | 17-02-1993 |
| | | | DE | 69113178 D1 | 26-10-1995 |
| | | | DE | 69113178 T2 | 21-03-1996 |
| | | | DK | 472291 T3 | 06-11-1995 |
| | | | EP | 0472291 A1 | 26-02-1992 |
| | | | ES | 2089138 T3 | 01-10-1996 |
| | | | FI | 913537 A | 25-01-1992 |
| | | | GR | 91100284 A ,B | 26-08-1992 |
| | | | HK | 20196 A | 09-02-1996 |
| | | | HU | 60550 A2 | 28-09-1992 |
| | | | IE | 912598 A1 | 29-01-1992 |
| | | | JP | 3022640 B2 | 21-03-2000 |
| | | | JP | 6201990 A | 22-07-1994 |
| | | | MX | 9100362 A1 | 28-02-1992 |
| | | | NO | 912880 A | 27-01-1992 |
| | | | NZ | 238960 A | 26-08-1993 |
| | | | PT | 98420 A | 29-10-1993 |
| | | | RO | 112931 B1 | 30-01-1998 |
| | | | SK | 229591 A3 | 09-08-1995 |
| | | | US | 5220359 A | 15-06-1993 |
| | | | ZA | 9105779 A | 31-03-1993 |
| US 5864378 | A | 26-01-1999 | EP | 0900404 A1 | 10-03-1999 |
| | | | JP | 2000511439 T | 05-09-2000 |
| | | | US | 6024447 A | 15-02-2000 |
| | | | WO | 9744698 A1 | 27-11-1997 |
| | | | US | 6126286 A | 03-10-2000 |

EPO FORM P0459

For more details about this annex : see Official Journal of the European Patent Office, No. 12/82

EP 1 402 852 A1

**ANNEX TO THE EUROPEAN SEARCH REPORT
ON EUROPEAN PATENT APPLICATION NO.**                EP 03 02 1678

This annex lists the patent family members relating to the patent documents cited in the above-mentioned European search report.
The members are as contained in the European Patent Office EDP file on
The European Patent Office is in no way liable for these particulars which are merely given for the purpose of information.

28-01-2004

| Patent document cited in search report | | Publication date | Patent family member(s) | | Publication date |
|---|---|---|---|---|---|
| WO 0184216 | A | 08-11-2001 | US | 6537317 B1 | 25-03-2003 |
| | | | AU | 5934601 A | 12-11-2001 |
| | | | BR | 0110514 A | 01-04-2003 |
| | | | CA | 2407891 A1 | 08-11-2001 |
| | | | EP | 1279062 A1 | 29-01-2003 |
| | | | JP | 2003531708 T | 28-10-2003 |
| | | | WO | 0184216 A1 | 08-11-2001 |
| US 4769033 | A | 06-09-1988 | US | 5326348 A | 05-07-1994 |
| | | | US | 2002183843 A1 | 05-12-2002 |
| | | | US | 4917681 A | 17-04-1990 |
| | | | US | 6007747 A | 28-12-1999 |
| | | | US | 5019099 A | 28-05-1991 |
| | | | US | 5236452 A | 17-08-1993 |
| | | | US | 5074877 A | 24-12-1991 |
| EP 0503111 | A | 16-09-1992 | EP | 0503111 A1 | 16-09-1992 |
| | | | US | 5191366 A | 02-03-1993 |
| US 5019098 | A | 28-05-1991 | FR | 2647227 A1 | 23-11-1990 |
| | | | DE | 69002386 D1 | 02-09-1993 |
| | | | DE | 69002386 T2 | 13-01-1994 |
| | | | EP | 0398813 A1 | 22-11-1990 |
| | | | JP | 3011315 A | 18-01-1991 |
| US 5888122 | A | 30-03-1999 | AU | 6957898 A | 30-10-1998 |
| | | | EP | 1011537 A1 | 28-06-2000 |
| | | | WO | 9844875 A1 | 15-10-1998 |
| US 5173723 | A | 22-12-1992 | EP | 0504393 A1 | 23-09-1992 |
| | | | WO | 9206400 A1 | 16-04-1992 |

EPO FORM P0459

For more details about this annex : see Official Journal of the European Patent Office, No. 12/82

17